# EUROPEAN PATENT APPLICATION

(11) **EP 4 755 365 A1**
(43) Date of publication of application: **10.06.2026**
(21) Application number: 25213095.0
(22) Date of filing: 03.11.2025
(51) Int. Cl.: A61H 1/02, A61B 5/00, A61B 5/11, A61H 3/00

(54) **GAIT REHABILITATION ROBOT AND TRAINING METHOD THEREOF**

(30) Priority: 07.11.2024 KR 20240157167
(71) Applicant: Curexo, Inc., Seoul 05814 (KR); The Board of Regents of the University of Texas System, Austin, TX 78701 (US)
(72) Inventor: HWANG, Sun Hee, 05814 Seoul (KR); MOON, Jun Sik, 05814 Seoul (KR); YOUN, Jae Woong, 05814 Seoul (KR); KIM, Young Hwan, 05814 Seoul (KR); KO, Man Soo, 41562 Daegu (KR); BROWN, David A., League City, 77573 (US)
(74) Representative: Zimmermann, Tankred Klaus

(57) **Abstract**

Disclosed are a gait rehabilitation robot and gait rehabilitation robot and training method thereof, the gait rehabilitation robot including: a pair of footplates; a pair of footplate supports; a pair of gait actuators; a controller; and a memory, wherein the controller receives at least one of a state value and a control command value relating to the corresponding motor from each gait actuator under a load condition, and calculates anteroposterior forces exerted on each of the footplates by the corresponding foot during the gait training based, for each gait actuator, on a difference between at least one of the received state value and control command value and the corresponding reference value stored in the memory for a present gait trajectory and speed. Thus, a gait status can be determined based on the difference between the anteroposterior forces exerted on each footplate under no-load and load conditions.

## Description

### BACKGROUND

### Field

The disclosure relates to a gait rehabilitation robot and a training method thereof and, more particularly, to a method of providing feedback to a trainee (e.g. patient) based on a horizontal force exerted on a footplate by the trainee, thereby enabling the trainee to actively participate in training.

### Description of the Related Art

In general, a gait rehabilitation robot refers to a robot used for gait rehabilitation therapy or the like, and is utilized in upright sensory enhancement training for patients with lower-limb paralysis caused by spinal cord injury, stroke, traumatic brain injury, muscular dystrophy, Parkinson's disease, multiple sclerosis, cerebral palsy, etc.

Various types of gait rehabilitation robots, such as treadmill-type, end-effector-type, and exoskeleton-type robots, are being used. In a fully automated system like the treadmill-type or end-effector-type robot, it is necessary to monitor the gait status of a trainee.

Conventionally, there have been cases where the gait rehabilitation robot employs a method of installing a load cell or similar force sensor on a footplate to detect vertical ground reaction forces and provide feedback to a patient. However, a patient's gait ability, such as step length and gait speed, is closely associated with horizontal ground reaction forces. Therefore, providing feedback on the vertical ground reaction forces caused by the shift in the center of mass makes it difficult to achieve effective gait training.

### SUMMARY

To solve the problems described above, an aspect of the disclosure is to estimate anteroposterior forces exerted on a footplate of a gait rehabilitation robot by a trainee and provide feedback, thereby enhancing the effectiveness of gait training. Further, when the estimation of the anteroposterior forces is completed, a threshold value for the force to be generated can be set based on a user's motor function level and transmitted back as a real-time image.

According to an embodiment of the disclosure, a gait rehabilitation robot includes: a pair of footplates configured to place left and right feet of a trainee thereon for gait training; a pair of footplate supports, each connected to a corresponding footplate; a pair of gait actuators configured to actuate respective footplates and respective footplate supports, each gait actuator comprising a motor for moving the corresponding footplate in anterior and posterior directions, and a motor driver for controlling the motor; a controller configured to control the pair of gait actuators to move and rotate the respective footplates based on a preset gait trajectory and speed; and a memory configured to store reference values for at least one of state and control of each of the motors according to the preset gait trajectory and speed, wherein the controller receives at least one of a state value and a control command value relating to the corresponding motor from each gait actuator under a load condition, and calculates anteroposterior forces exerted on each of the footplates by the corresponding foot during the gait training based, for each gait actuator, on a difference between at least one of the received state value and control command value and the corresponding reference value stored in the memory for a present gait trajectory and speed.

In addition, the reference values may include at least one of a current value, a torque value, a position value, a speed value, a current command value, a force command value, a position command value, and a speed command value of the motor under a no-load condition, the state value may comprise at least one of a current value, a torque value, a position value, and a speed value of the motor, and the command value comprises at least one of a current command value, a position command value, a speed command value, and a torque command value.

Further, the gait actuator may further include a current sensor configured to sense a current of the motor; the reference values of the motor may include a first current value of the motor measured by the current sensor under the no-load condition or a first torque value calculated based on the first current value; the state value may include a second current value of the motor measured by the current sensor under the load condition or a second torque value calculated based on the second current value; and the controller may calculate the anteroposterior forces exerted on each of the footplates by the corresponding foot, based on a difference between the second current value or second torque value of the corresponding motor under the load condition and the first current value or first torque value of the corresponding motor under the no-load condition.

In addition, the reference values of the motor may include a first current command value or first torque command value applied by the motor driver under the no-load condition; the command value may a second current command value or second torque command value applied by the motor driver under the load condition; and the controller may calculate the anteroposterior forces exerted on each of the footplates by the corresponding foot, based on a difference between the second current command value or second torque command value of the corresponding motor under the load condition and the first current command value or first torque command value of the corresponding motor under the no-load condition.

In addition, the gait actuator may further include an encoder connected to the motor and configured to detect a position and speed of the motor; the reference values of the motor may include a first position value or first speed value of the motor under the no-load condition; the state value may include a second position value or second speed value of the motor, calculated by the encoder under the load condition; and the controller may calculate the anteroposterior forces exerted on the footplate by the feet, based on a difference between the first position value or first speed value and the first position value or second position value.

Further, the controller may generate and provide training feedback information related to a gait training status of the trainee based on comparison between the anteroposterior forces of a reference-side footplate in a first step and a training-side footplate in a second step, and the training feedback information may include at least one of visual feedback information, auditory feedback information, and tactile feedback information.

In addition, the reference values may include state values or control values of the motor acquired according to a plurality of different gait trajectories and a plurality of different speeds by controlling each gait actuator to operate along the plurality of different gait trajectories and at the plurality of different speeds under the no-load condition, or comprises a control value of state value of the motor determined by a robot model.

Furthermore, the controller may synchronize at least one of the state value and command value of each motor with the reference values according to gait cycles, and calculates differences between at least one of the synchronized state value and command value and the reference values throughout the gait cycle.

Further, the gait rehabilitation robot may further include a user interface (UI) generator configured to generate a first UI menu for setting a target range of anteroposterior forces exerted on a training-side footplate by the trainee compared to the anteroposterior forces exerted on a reference-side footplate, wherein the controller may compare absolute values of anteroposterior forces exerted on the reference-side footplate in a first step with absolute values of anteroposterior forces exerted on the training-side footplate in a second step, and determines that the gait training of the trainee is in a normal state upon the anteroposterior forces exerted on the training-side footplate falling within the target range, but determines that the gait training is in an abnormal state upon the anteroposterior forces exerted on the training-side footplate falling outside the target range.

Further, the gait rehabilitation robot may further include: a user interface (UI) generator configured to generate a first UI menu for setting a target range of anteroposterior forces exerted on a training-side footplate by the trainee compared to the anteroposterior forces exerted on a reference-side footplate; and a display, wherein the controller may control the UI generator to generate a second UI menu that represents the magnitudes of anteroposterior forces exerted on the reference-side footplate and the training-side footplate with respect to a target range and to display the second UI menu on the display.

In addition, the gait rehabilitation robot may further include: a user interface (UI) generator configured to generate a first UI menu for setting a target value of anteroposterior forces exerted on the training-side footplate by the trainee compared to the anteroposterior forces exerted on the reference-side footplate; wherein the training feedback information includes virtual reality-based feedback information corresponding to each of a plurality of preset training states; and wherein the controller is further configured to calculate a difference between the anteroposterior forces exerted on the training-side footplate and the target value, and to generate and provide virtual reality-based feedback information corresponding to one of the plurality of the training states based on the calculated difference

In addition, the gait rehabilitation robot may further include a loudspeaker, wherein the controller generates an audio feedback signal that indicates timing of the anteroposterior forces to be exerted on the training-side footplate and controls the loudspeaker to output the audio feedback signal, upon the anteroposterior forces exerted on the training-side footplate falling outside the target range or upon timing of the anteroposterior forces exerted on the training-side footplate being incorrect.

Further, the controller may correct at least one of a gait trajectory and speed for at least one of the reference-side footplate and the training-side footplate based on a difference in the anteroposterior forces between the reference-side footplate and the training-side footplate or a difference between the anteroposterior forces of the training-side footplate and the target value.

Meanwhile, according to an embodiment of the disclosure, a gait rehabilitation robot includes: a pair of footplates configured to place left and right feet of a trainee thereon for gait training; a pair of footplate supports, each connected to a corresponding footplate; a pair of gait actuators configured to actuate respective footplates and respective footplate supports, each gait actuator comprising a motor for moving the corresponding footplate in anterior and posterior directions, an encoder connected to the motor and detecting position and speed of the motor, and a motor driver for controlling the motor; a controller configured to control the pair of gait actuators to move and rotate the respective footplates based on a preset gait trajectory and speed; and a memory configured to store reference values for position or speed of the motor according to the preset gait trajectory and speed, wherein the motor driver calculates a feedback command value based on an error by comparing a position value or speed value of a corresponding motor calculated by the encoder under the load condition with the reference values, and applies the feedback control value to the corresponding motor, and the controller calculates anteroposterior forces exerted on each of the footplates by the corresponding foot during the gait training based on the feedback command value.

Meanwhile, according to an embodiment of the disclosure, a training method of a gait rehabilitation robot including a pair of footplates configured to place left and right feet of a trainee thereon for gait training, and a motor for moving each footplate in anterior and posterior directions, includes: storing, for each motor, reference values for at least one of a control or a state of the motor based on a preset gait trajectory and speed; acquiring at least one of a state value and control command value of each motor under a load condition; and calculating anteroposterior forces exerted on each footplate by the corresponding foot during the gait training, based, for each motor, on a difference between at least one of the state value and command value and the corresponding reference values corresponding to a present gait trajectory and speed.

Further, the training method may further include generating and providing training feedback information related to a gait training status of the trainee based on comparison between the anteroposterior forces of a reference-side footplate and a training-side footplate, wherein the training feedback information comprises at least one of visual feedback information, auditory feedback information, and tactile feedback information.

Furthermore, the training method may further include: setting a target value for the anteroposterior forces exerted on the training-side footplate; and wherein the training feedback information includes virtual reality-based feedback information corresponding to each of a plurality of preset training states; and wherein the generating and providing the training feedback information comprises calculating a difference between the anteroposterior forces exerted on the training-side footplate and the target value, and generating and providing virtual reality-based feedback information corresponding to one of the plurality of the training states based on the calculated difference.

In addition, the calculating the anteroposterior forces comprises synchronizing at least one of the state value and command value of the motor with the reference values according to gait cycles.

Furthermore, the training method may further include: generating and providing a first UI menu for setting a target range of anteroposterior forces exerted on a training-side footplate by the trainee compared to the anteroposterior forces exerted on a reference-side footplate; and generating and providing a second UI menu that represents the magnitudes of anteroposterior forces exerted on the reference-side footplate and the training-side footplate with respect to a target range.

Further, the training method may further include: setting a target value for the anteroposterior forces exerted on a training-side footplate; and correcting at least one of a gait trajectory and speed for at least one of a reference-side footplate and the training-side footplate based on a difference in the anteroposterior forces between the reference-side footplate and the training-side footplate, or a difference between the anteroposterior forces of the training-side footplate and the target value.

### BRIEF DESCRIPTION OF THE DRAWINGS

FIG. 1 is a schematic diagram of a gait rehabilitation robot according to an embodiment of the disclosure.
FIG. 2 is a schematic diagram of a gait actuator in a gait rehabilitation robot according to an embodiment of the disclosure.
FIG. 3 is a perspective view illustrating a structure of a gait rehabilitation robot according to an embodiment of the disclosure.
FIG. 4 is a flowchart showing a control method of a gait rehabilitation robot according to an embodiment of the disclosure.
FIG. 5 is a flowchart showing a control method of a gait rehabilitation robot according to an embodiment of the disclosure.
FIG. 6A shows torque values of a motor over time under no-load condition, which are estimated by a controller according to an embodiment of the disclosure.
FIG. 6B shows multiple torque values of the motor under the no-load condition of FIG. 6A, which are divided in units of gait cycles and arranged according to the respective gait cycles.
FIG. 6C shows average torque values of the motor over gait cycles under the no-load condition of FIG. 6B.
FIG. 7A shows torque values of a motor over time under load condition, which are estimated by a controller according to an embodiment of the disclosure.
FIG. 7B shows first torque (solid line) under the no-load condition and second torque (dotted line) under the load condition, and FIG. 7C shows a difference between the first torque and the second torque.
FIGS. 8A and 8B show anteroposterior forces exerted on left and right footplates in a gait training robot according to an embodiment of the disclosure.
FIG. 9 shows an example of a feedback screen displaying a gait training status of a gait training robot according to an embodiment of the disclosure.
FIGS. 10A, 10B and 10C show three examples of a virtual reality feedback screen displaying different gain training statuses of the gain training robot according to an embodiment of the disclosure.

### DETAILED DESCRIPTION OF EMBODIMENTS

Below, specific embodiments of the disclosure will be described in detail with reference to the accompanying drawings.

Hereinafter, exemplary embodiments of the disclosure will be described in detail with reference to the accompanying drawings. However, detailed descriptions about known functions or configurations, which may obscure the gist of the disclosure, are omitted from the following descriptions and the accompanying drawings. Further, it is noted that like numerals refer to like elements throughout the accompanying drawings.

It should be understood that the terms used in this specification and the appended claims should not be construed as limited to typical and lexical meanings, but interpreted based on the meanings and concepts corresponding to technical aspects of the disclosure on the basis of the principle that the inventor is allowed to define terms appropriately for the best description. Therefore, the description proposed herein is merely a preferable example for the illustrative purpose only, not intended to limit the scope of the disclosure, so it should be understood that other equivalents and modifications could be made thereto without departing from the spirit and scope of the disclosure.

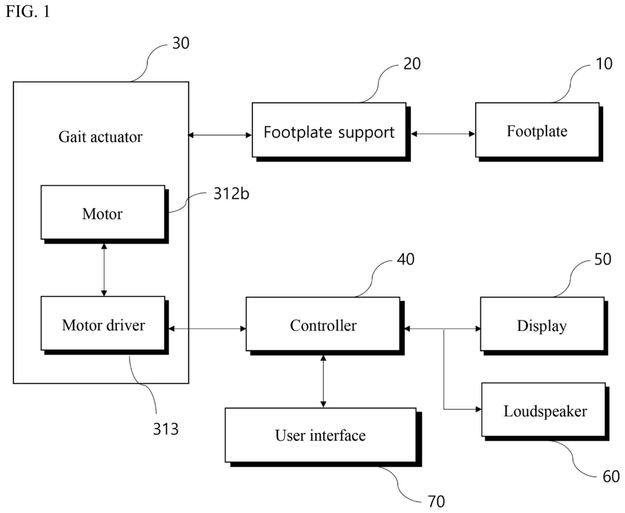

Hereinafter, exemplary embodiments of the disclosure will be described in detail with reference to FIGS. 1 to 10, in which like numerals refer to like elements throughout. Meanwhile, in the accompanying drawings, illustrations and detailed descriptions about the configurations, operations, and effects, which can be readily understood by those skilled in the art from general technical knowledge, will be simplified or omitted, and the description will focus on parts related to the disclosure.

FIG. 1 is a schematic diagram of a gait rehabilitation robot according to an embodiment of the disclosure.

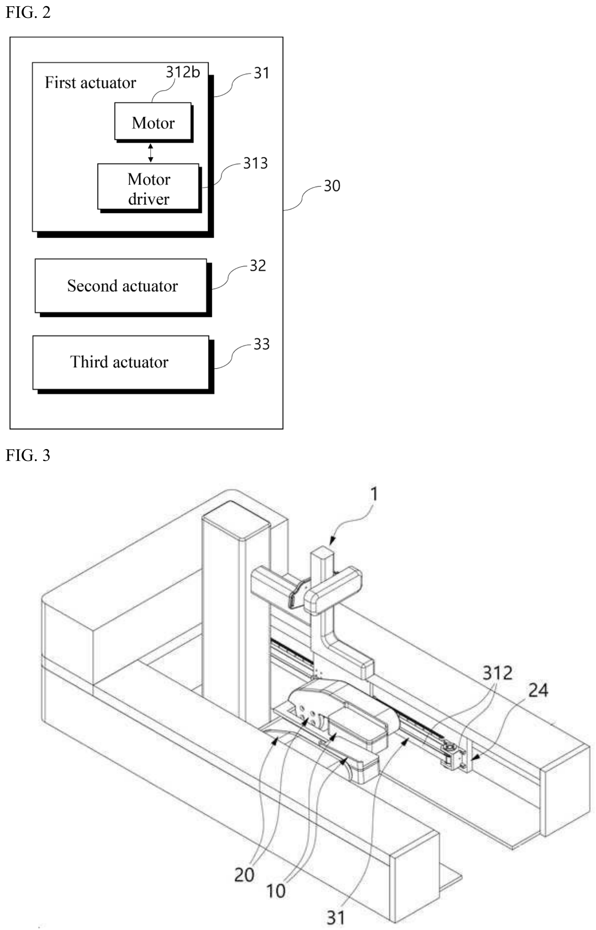

Referring to FIGS. 1 and 2, the gait rehabilitation robot according to an embodiment of the disclosure includes a pair of footplates 10 on which a trainee can place his/her left and right feet for gait training, a pair of footplate supports 20 to which the respective footplates 10 are connected, a pair of gait actuators 30 for actuating the respective footplates 10 and the respective footplate supports 20, and a controller 40 for controlling the pair of gait actuators 30. In FIG. 1, for convenience of description only one footplate (10), one footplate support (20), and one gait driving unit (30) are illustrated; however, it should be noted that each of these is provided on both the left and right sides, i.e., two in total.

The footplates 10 are provided on the left and right sides so that both feet of the trainee can be stably placed thereon and undergo the gait training, and may be configured in various ways without any specific restrictions on their shapes or structures as long as the structure allows for easy separation of the trainee's feet in case of spasticity of the trainee or abnormal operations of the gait rehabilitation robot.

The footplate supports 20 are connected to the respective footplates 10 to support the footplates 10. The footplate supports 20 are provided for the left and right footplates 10, respectively.

The gait actuators 30 drive the respective footplates 10 and respective footplate supports 20 to move and rotate under control of the controller 40 (to be described later) in order to implement a gait trajectory. Each gait actuator 30 may include a first actuator 31 for translational movement of the footplate 10 in anterior and posterior directions, and may further include a second actuator 32 for rotational movement of the footplate support 20, and a third actuator 33 for rotational movement of the footplate 10.

The first actuator 31 includes a motor 312b for the translational movement of the corresponding footplate 10 in the anterior and posterior directions, and a motor driver 313 for the control of the motor 312b.

The motor driver 313 is to drive the motor 312b based on a preset control algorithm, for example, to control the position, speed, current, and/or torque of the motor 312b. The motor driver 313 may be implemented by a control algorithm, a control circuit, a microcontroller, and/or a processor according to the specifications of the motor 312b or a predetermined robot model.

According to an embodiment of the disclosure, the first actuator may further include a current sensor for sensing the current of the motor 312b. The current sensor may include a resistor or the like to detect the output current of the motor 312b. The motor driver 313 may receive a detected current value from the current sensor and calculate a torque value of the motor 312b based on the received current value. The motor driver 313 may use a torque constant of the motor 312b to calculate the torque value based on the current value.

According to an embodiment of the disclosure, the first actuator may further include an encoder that is connected to the motor 312b and detects the position and speed of the motor 312b. The motor driver 313 may receive a position value or speed value detected by the encoder, compare the received value with a preset target position value (position command value) or target speed value (speed command value), and calculate a command value (current command value, torque command value) for feedback control, which minimizes the errors (e.g. the difference between the compared values), and apply the calculated command value to the motor 312b.

The controller 40 is to control the gait actuators 30 to move and rotate the respective footplates 10 based on the preset gait trajectory and speed, and analyze a gait based on a state or command value for the state or control of each motor 312b. The controller 40 includes a memory for storing control program codes and data, and a processor for executing the control program codes.

The memory refers to a recording medium readable by a computing device, and may include a nonvolatile memory such as a read only memory (ROM), an erasable programmable ROM (EPROM), an electrically erasable programmable ROM (EEPROM), and a flash memory; a storage such as a hard disk drive (HDD), a solid state disk (SSD), and a detachable disk; a volatile memory such as a random access memory (RAM), or a recording medium readable by any type of computer well known in the art to which the disclosure pertains. The memory may store at least one computer program code to be executed by the processor. Such a computer program code may be loaded into the memory from a floppy drive, a disk, a memory card, etc., other than a built-in memory of a device. The memory may store software for analyzing a gait, and gait data of a trainee.

The processor is to execute and process computer program instructions by performing basic logic, calculations, and operations, and may include at least one of a central processing unit (CPU), a microprocessor unit (MPU), an microcontroller unit (MCU), a graphic processing unit (GPU), or any other form of processor well known in the art to which the disclosure pertains. The computer program code stored in the memory is loaded into the processor and executed. The processor may execute an algorithm stored in the memory to analyze a gait in real time, and further perform a series of functions for correcting the gait trajectory and speed.

Meanwhile, reference values for the state or control of the motor 312b operating under a no-load condition are stored in the memory. The reference values include at least one of the current value, torque value, position value, speed value, current command value, force command value, position command value, and speed command value of the motor 312b under the no-load condition.

According to an embodiment of the disclosure, the controller 40 may drive the gait actuators 30 according to the preset gait trajectory and speed under the no-load condition, thereby obtaining the reference values and storing the obtained reference values in the memory. The no-load condition means that no person is on the gait rehabilitation robot.

As described above, the current value may be detected by the current sensor; the torque value may be calculated by the motor driver 313; and the position value and the speed value may be calculated by the encoder. The current command value, the force command value, the position command value, and the speed command value are values applied from the motor driver 313 to the motor 312b and may be obtained through the motor driver 313.

In the gait rehabilitation robot according to the disclosure, the gait trajectory and speed are predetermined values depending on a training mode. Because the gait trajectory and speed vary depending on the training mode, the controller 40 may operate the gait actuator 30 in multiple training modes under the no-load condition to obtain the reference values for the state or control of the motor 312b according to each training mode, and store the obtained reference values in the memory.

According to another embodiment of the disclosure, the reference values may be control values or state values of the motor 312b, which are determined in advance according to the specifications of the motor 312b or the robot model. In this case, the reference values may be stored in the memory of the motor driver 313 or the memory of the controller 40 when manufacturing the gait rehabilitation robot, and may be updated by the controller 40.

The memory in which the reference values are stored may be involved in or be provided separately from the motor driver 313 or the controller 40.

The controller 40 receives at least one of a state value and a control command value for the state and control of the motors 312b from the gait actuators 30 under the load condition, and calculates the anteroposterior forces exerted on each of the footplates 10 by the feet during the gait training based on the difference between at least one of the received state value and command value and the reference values of the motor 312b corresponding to the current gait trajectory and speed.

The load condition means that the trainee is on the gait rehabilitation robot and undergoes the gait training.

The state value for the state of the motor 312b includes at least one of the position value, speed value, current value, and torque value of the motor 312b, and may be detected or calculated by the encoder, the current sensor, or the motor driver 313.

As described above, the current value may be detected by the current sensor, the motor driver 313 may calculate the torque value based on the current value, and the position value and the speed value may be calculated by the encoder. The controller 40 may receive the state values of the motor 312b through the current sensor, the encoder, and the motor driver 313.

According to another embodiment, when the motor driver 313 is connected to the current sensor and the encoder to control the motor 312b and the state values of the motor 312b are known, the controller 40 may receive the state values of the motor 312b, such as the current value, the torque value, the position value, and the speed value, through the motor driver 313.

According to another embodiment, the motor driver 313 may receive the position value or speed value of the motor 312b from the encoder, and calculate the current value or torque value of the motor 312b based on the received position or speed value. For example, the motor driver 313 may apply a target command value (position command value or speed command value) to the motor 312b to achieve the gait trajectory and speed according to the training mode during the gait training, receive the position value or speed value detected by the encoder, compare the received position value or speed value with the target command value, and calculate the current command value or the torque command value to compensate for the error (e.g. the difference between the compared values). Similarly, in the case of feedback control, the motor driver 313 applies the calculated current command value or torque command value to the motor 312b. The motor driver 313 may calculate the current value or torque value of the motor 312b under the load condition based on the target command value and the command value calculated to compensate for the error, and then transmit the calculated current value or torque value of the motor 312b to the controller 40.

The controller 40 receives the state value for state and/or the command value for control of the motors 312b obtained as above, and compares the received value with the corresponding reference values stored in the memory, thereby calculating the anteroposterior forces exerted on each of the footplates 10 by the trainee's feet under the load condition.

Hereinafter, for clarity of description, a first state value, a first current value, a first torque value, a first position value, a first speed value, a first command value, a first current command value, a first torque command value, a first position command value, and a first speed command value of the motor 312b will be referred to as values related to the state or control of the motor 312b under the no-load condition, and a second state value, a second current value, a second torque value, a second position value, a second speed value, a second command value, a second current command value, a second torque command value, a second position command value, and a second speed command value will be referred to as values related to the state or control of the motor 312b under the load condition.

According to a first embodiment, the controller 40 may receive the second current value or the second torque value from the current sensor or the motor driver 313 under the load condition, and compare the second current value (second torque value) with the first current value (or first torque value) stored in the memory, thereby calculating the anteroposterior forces exerted on each of the footplates 10 by the foot based on a difference between the compared values.

According to a second embodiment, the controller 40 may receive the second position value or the second speed value from the encoder or the motor driver 313 under the load condition, and compare the received second position or speed value with the first position value (or first speed value) stored in the memory, thereby calculating the anteroposterior forces exerted on the footplate 10 by the foot based on a difference between the compared values. The controller 40 may calculate the current value (or torque value) based on the error (e.g. difference) between the reference value under the no-load condition and the state value under the load condition, thereby calculating the anteroposterior forces exerted on each of the footplates 10 by the foot based on the calculated current value (torque value).

According to a third embodiment, the controller 40 may receive the second current command value (or second torque command value) or the second position command value (or second speed command value) from the motor driver 313 under the load condition, and compare the received value with the first current command value (or first torque command value) or the first position command value (or first speed command value) stored in the memory, thereby calculating the anteroposterior forces exerted on each of the footplates 10 by the foot based on differences between the compared values.

According to a fourth embodiment, the controller 40 may receive a command value (current command value or torque command value) for the feedback control from the motor driver 313 under the load condition, and calculate the anteroposterior forces exerted on the footplate 10 by the foot based on the command value for the feedback control. Because the command value for the feedback control is intended to compensate for the error between the current state value of the motor 312b and the target command value, the command values may be estimated as the anteroposterior forces exerted on the footplate 10 by the foot.

The controller 40 generates and provides training feedback information about a gait training status based on a difference in the anteroposterior forces between the reference-side footplate 10 in a first step and the training-side footplate 10 in a second step. The training feedback information includes at least one of visual feedback information, auditory feedback information, and tactile feedback information.

The controller 40 may provide visual, auditory, or tactile feedback to a trainee based on the difference in the anteroposterior forces between the reference-side footplate 10 in the first step and the training-side footplate 10 in the second step, thereby guiding training so that the reference-side and training-side anteroposterior forces can become symmetrical, and generating and providing feedback information about timing, at which the anteroposterior forces should be generated, to the trainee.

According to an embodiment of the disclosure, the gait rehabilitation robot may further include a display 50 for displaying data and images, a loudspeaker 60 for outputting audio, and a user interface 70 for receiving a user's input. The user interface 70 may for example include an input device such as a microphone, a keyboard, or a mouse. Further, the robot may include a vibration sensor for providing tactile feedback information to a trainee.

Meanwhile, the controller 40 may include a user interface (UI) generator that generates a first UI menu for setting a target range of the anteroposterior forces that a trainee should generate on the training-side footplate 10. Here, the target range refers to the magnitude or ratio of the anteroposterior forces of the training-side footplate 10 to the anteroposterior forces of the reference-side footplate 10, and a user may select a target range within a range of 30 to 100% on the first UI menu. The target range may be set by a user, i.e., a therapist, and may be appropriately set according to the gait health conditions of a trainee. For example, when the user selects 50% on the first UI menu, the controller 40 sets a range of 50% to 100% as the target range. According to another embodiment of the present invention, the first UI menu of the UI generator may further include an item for setting a target value for the anteroposterior forces to be applied to the training-side footplate 10.

The UI generator may generate a second UI menu that shows comparison between the magnitudes of the anteroposterior forces exerted on the reference-side footplate 10 and the training-side footplate 10. The controller 40 generates the second UI menu that visually represents the magnitudes of the anteroposterior forces exerted on the reference-side footplate 10 and the training-side footplate 10 during the gait training and displays the second UI menu on the display 50 so that a trainee or a therapist can check the magnitudes of the anteroposterior forces. Further, the second UI menu may display the anteroposterior forces exerted on the reference-side footplate 10 and the training-side footplate 10 in different colors, and furthermore, may display the target range of the anteroposterior forces of the training-side footplate 10, thereby visually indicating whether the anteroposterior forces currently exerted on the training-side footplate 10 is within, below or above the target range. This allows the trainee or therapist to know whether the forces exerted on the training-side footplate 10 is appropriate or needs to be increased, thereby enabling the training to be carried out more thoroughly.

In another embodiment of the disclosure, when the anteroposterior forces exerted on the training-side footplate 10 deviate from the set target range or the timing of the anteroposterior forces exerted on the training-side footplate 10 deviates from normal timing, the controller 40 may generate an audio feedback signal indicating the timing of the anteroposterior forces to be exerted on the training-side footplate 10 and control the loudspeaker to output the audio feedback signal, or may generate a tactile feedback signal and control the vibration sensor to output the tactile feedback signal. This allows a trainee to know the timing at which he or she should apply force to his/her training-side foot, thereby helping the gait training to be performed normally.

FIG. 3 is a perspective view showing the structure of a gait rehabilitation robot according to an embodiment of the disclosure. Although FIG. 3 illustrates an end-effector type robot as an example of the gait rehabilitation robot, it should be noted that the disclosure is not limited thereto and may be applied to various footplate-type gait rehabilitation devices such as a treadmill type robot.

Referring to FIG. 3, the gait rehabilitation robot according to an embodiment of the disclosure includes a weight support 1 for supporting the weight of a trainee, such as a trainee in need of gait rehabilitation training, s pair of footplates 10 and a pair of footplate supports 20 arranged symmetrically on both sides of the weight support 1 so that the trainee riding the weight support 1 can actually undergo the gait rehabilitation training, and the gait actuators 30 for moving and rotating the respective footplates 10 and respective footplate supports 20.

Each footplate support 20 is formed as an arm-shaped structure having a first end connected to a transfer mechanism (to be described later) and a second end to which the footplate 10 is mounted and includes a support link connected to an output end of a support reduction device and a support housing coupled to the support link.

Each gait actuator 30 includes the first actuator 31 for the translational movement of the footplate support 20 in the anterior and posterior directions, the second actuator 32 for the rotational movement of the footplate support 20, and the third actuator 33 for the rotational movement of the footplate 10. Here, an actuator suspending member 24 is provided so that the first actuator 31 can be installed being suspended on the side rather than being placed on a structure installed on the floor.

Regarding the structure of the end-effector type gait rehabilitation robot, similar structures have been described in Korean Patent Nos. 10-1623686 and 10-2127011, etc., and thus the detailed descriptions thereof will be omitted. Below, only the first actuator 31 for the translational movement of the footplate 10 will be described in more detail.

The first actuator 31 refers to an actuator for the movement of the footplate support 20 in the anterior and posterior directions, and may include the transfer mechanism to which the footplate support 20 is connected, and a transfer actuator 312 that applies a driving force to the transfer mechanism.

The transfer mechanism may include a guide rail installed in a suspended form along the direction of translational movement, a slider connected to the guide rail, and a transfer base on which the slider and the footplate support 20 are installed. Here, the guide rail and the slider may be selected and applied without any specific restrictions as long as they are linear motion guiding means to effectively guide a linear motion.

The transfer actuator 312 may include a band-shaped transfer belt having both ends fixed to a vertical base of the transfer base by fixing brackets and installed to move along an arrangement path of the guide rail, the motor 312b generating and providing the driving force for the movement of the transfer belt, a transfer reduction device connected to a motor shaft of the motor 312b and performing a deceleration function, a transfer drive pulley installed at an output end of the transfer drive pulley and engaging with a first side of the transfer belt, and a transfer driven pulley spaced apart from the transfer drive pulley and engaging with a second side of the transfer belt. In addition, the motor 312b is coupled to the actuator suspending member 24 by a coupling bracket, and includes a motor shaft on which a motor-side power transmission pulley is installed. The transfer reduction device is coupled to the actuator suspending member 24 by the coupling bracket and includes a reduction-side power transmission pulley on which a second side of a belt (not shown) having a first side engaging with the motor-side power transmission pulley and an output end to which the transfer drive pulley is installed. The transfer driven pulley may be rotatably installed on the coupling bracket coupled to the actuator suspending member 24.

When the first actuator 31 drives the motor 312b, the rotational force of the motor-side power transmission pulley is transmitted to the reduction-side power transmission pulley via a belt (not shown), and the rotational force is reduced by the transfer reduction device, output to the transfer drive pulley, and transmitted to the transfer belt wound between the transfer drive pulley and the transfer driven pulley. Accordingly, when the transfer belt performs forward and reverse orbital motions, the transfer base fixed to the transfer belt via the fixing bracket moves anteriorly and posteriorly, and the footplate support 20 including the footplate 10 coupled to the transfer base moves anteriorly and posteriorly.

The transfer actuator 312 refers to a component that applies a driving force for the movement of the slider (not shown). Besides the foregoing belt drive method, the transfer actuator 312 may also be achieved by a servo motor (not shown) mounted on the guide rail and generating a driving force, and a ball screw (not shown) connected to the servo motor and rotating to move the slider anteriorly and posteriorly.

The motor driver 313 (FIG. 2) may apply a command value for controlling the operation of the motor 312b of the first actuator, and the command value applied from the motor driver 313 may be monitored by the controller 40. Further, according to an embodiment of the disclosure, the motor driver 313 may continuously or periodically calculate the torque value of the motor 312b and transmit the calculated values to the controller 40.

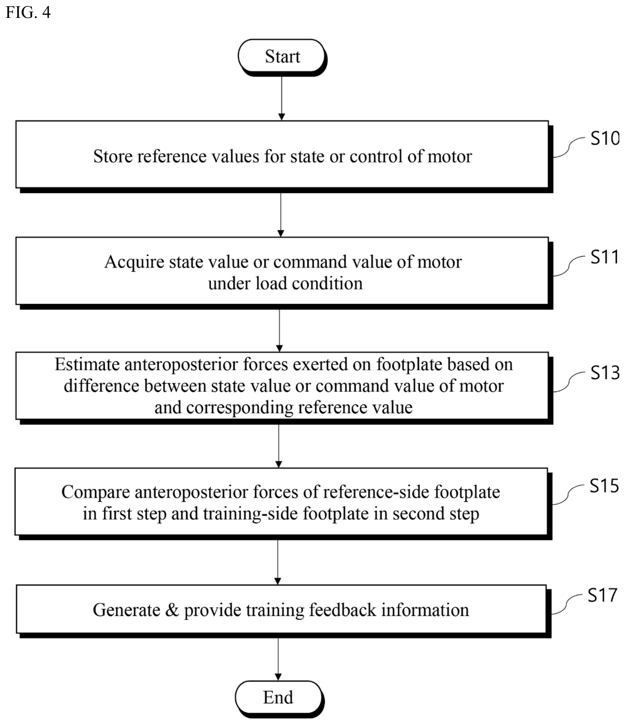

Hereinafter, a control method of a gait rehabilitation robot according to an embodiment of the disclosure will be described with reference to FIG. 4. FIG. 4 is a flowchart showing the control method of the gait rehabilitation robot according to an embodiment of the disclosure.

The controller 40 drives the gait rehabilitation robot under the no-load condition, and obtains the reference values for the state or control of the motor 312b of the first actuator 31 to stores the reference values in the memory (S10). The reference values may include the first current value detected from the current sensor, the first torque value calculated according to the first current value, or the first position value or first speed value detected by the encoder, and may include the first current command value or first torque command value, and the first position command value or first speed command value applied from the motor drivers 313. As described above, the reference values may also be calculated by the predetermined specifications of each motor 312b and the robot model.

The reference values may be acquired continuously or periodically according to gait cycles and may be acquired repeatedly in units of gait cycles and then averaged and set as the reference values.

The controller 40 acquires the second state value or second command value of each gait actuator 30, e.g., each motor 312b from the corresponding motor driver 313 under the load condition (i.e., during the gait training) (S11), compares the acquired value with the corresponding reference values and estimates the anteroposterior forces exerted on the footplate 10 based on differences between the compared values(S13).

The second state value under the load condition may include the second current value detected from the current sensor, the second torque value calculated based on the second current value, or the second position value or second speed value detected by the encoder, and may include the second current command value or second torque command value, and the second position command value or second speed command value applied from the motor driver 313.

The controller 40 synchronizes the second state value or second command value of each motor 312b acquired under the load condition with the corresponding reference values based on the gait cycle and calculates differences between the reference values and the second state values or second command values during the training. Accordingly, the anteroposterior forces exerted on each footplate 10 by the foot are calculated as continuous values over time.

The controller 40 compares the anteroposterior forces of the reference-side footplate 10 in the first step and the training-side footplate 10 in the second step (S15) and generates and provides training feedback information based on the comparison result (S17).

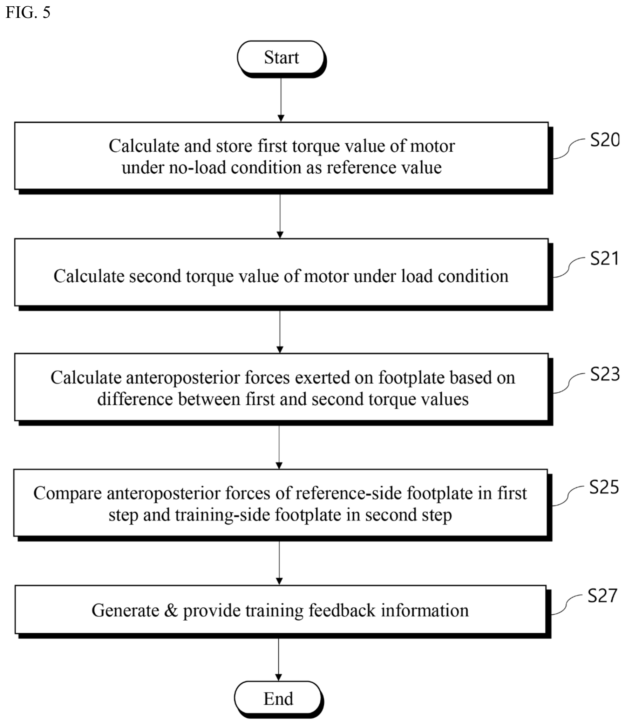

Hereinafter, a control method of a gait rehabilitation robot according to an embodiment of the disclosure will be described with reference to FIGS. 5 to 9. In the following embodiment, it will be described by way of example that the motor driver 313 calculates the torque value of the motor 312b based on the current value detected from the current sensor or the position value or speed value detected from the encoder.

FIG. 5 is a flowchart showing a control method of a gait rehabilitation robot according to an embodiment of the disclosure.

The controller 40 drives the gait rehabilitation robot under the no-load condition, and the motor driver 313 calculates the first torque value of the motor 312b (S20). The no-load condition means that the trainee is not on the gait training device, that the controller 40 controls the gait actuator 30 to be driven in a plurality of gait training modes according to a preset gait training program under the no-load condition, and that the motor driver 313 calculates the torque value of the motor 312b periodically or continuously. The controller 40 receives the torque values of the motor 312b according to the gait training modes calculated by the motor driver 313 and stores the calculated torque values as the reference values, i.e., the first torque values.

Specifically, under the control of the controller 40, the motor 312b of the first actuator 31 is operated to perform the translational movement of the footplate support 20 in the anterior and posterior directions; the motor of the second actuator 32 is operated to perform the rotational movement of the footplate support 20; and the motor 312b of the third actuator 33 is operated to perform the rotational movement of the footplate 10. In this case, the controller 40 regards the torque value of the motor 312b transmitted from the motor driver 313 as the first torque value of the no-load condition.

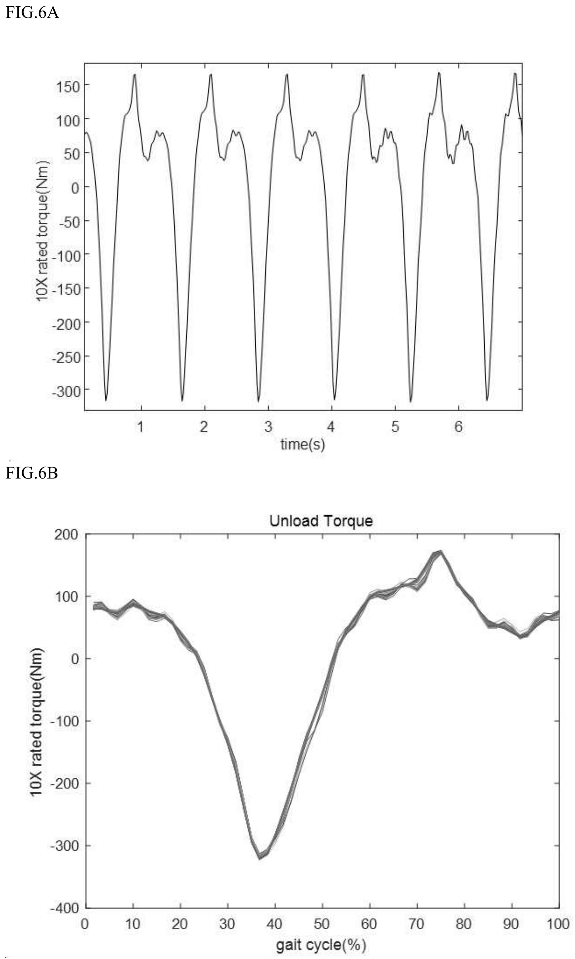

FIG. 6A shows the torque value of the motor 312b over time under the no-load condition, calculated by the motor driver 313 according to an embodiment of the disclosure, in which the motor 312b under the no-load condition exhibits a consistent torque value in accordance with the gait cycle.

The controller 40 may directly utilize the torque value of the motor 312b to calculate the anteroposterior forces exerted on the footplate 10, or may estimate or directly measure the current value based on the torque value of the motor 312b calculated from the motor driver 313 and calculate the anteroposterior forces based on the estimated or directly measured current value. In the following embodiments, it will be described by way of example that the controller 40 directly utilizes the torque value calculated by the motor driver 313.

FIG. 6B shows multiple torque values of the motor 312A under the no-load condition of FIG. 6A, which are divided in units of gait cycles and arranged according to the respective gait cycles. Because the controller 40 stores gait cycle data according to the gait training program, the torque value of the motor 312b calculated by the motor driver 313 may be divided or arranged according to the gait cycles as shown in FIG. 6B.

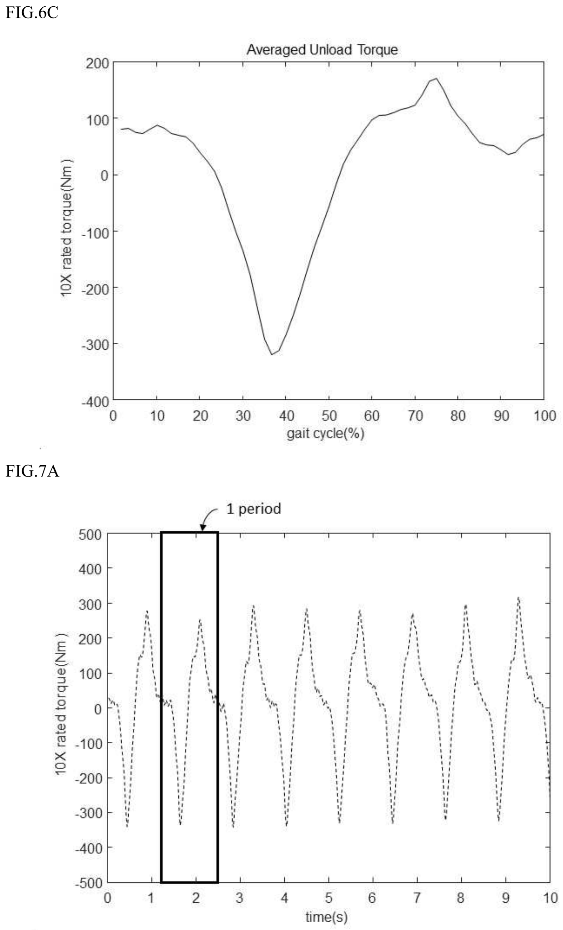

FIG. 6C shows average torque values of the motor 312b over gait cycles under the no-load condition of FIG. 6B, and the controller 40 determines the average torque values as the first torque value of the no-load condition.

Here, the gait program divides and averages the torque values of the motor 312b in units of gait cycles only for the anteroposterior forces corresponding to the same gait cycle or gait trajectory.

Meanwhile, the torque value of the motor 312b varies depending on the gait trajectory and speed. Therefore, when the gait training program has multiple gait training modes according to various gait trajectories and the gait speeds, the controller 40 operates the motor 312b in the multiple gait training modes, calculates the first torque values of the motor 312b corresponding to various gait trajectories and gait speeds, and stores the calculated first torque values in the memory as a data table.

The controller 40 drives the gait rehabilitation robot under the load condition according to the gait training program selected by a user during the gait training, and the motor driver 313 calculates the torque value of the motor 312b of the first actuator 31 (S21).

The load condition means that the trainee is riding the gait training robot and undergoing the gait training, and the controller 40 controls the gait actuator 30 to operate according to the preset gait training program while the trainee is riding the gait training robot and regards the torque value of the motor 312b of the first actuator 31 calculated by the motor driver 313 as the torque value of the motor 312b under the load condition, i.e., the second torque.

Referring to FIG. 3, when a trainee wants to undergo the gait training, s/he moves to the entrance of an entry space between cover members, where the left and right gait actuators 30 are located, while being seated in a wheelchair, stops at a position close to the footplates 10, rides with his/her hip positioned on a saddle, and places and secures his/her feet on the footplates 10 of the gait actuators 30, thereby undergoing the gait rehabilitation by the gait actuator 30 operating under the control of the controller 40 according to the gait training program selected by him/her.

When a trainee is seated on a seat and his/her feet are properly positioned on the footplates 10, each of the gait actuators 30 operates under the control of the controller 40 in response to an input signal so that the motor 312b of the first actuator 31 can perform the translational movement of the footplate support 20 in the anterior and posterior directions, the motor 312b of the second actuator 32 can perform the rotational movement of each footplate support 20, and the motor 312b of the third actuator 33 can perform the rotational movement of each footplates 10, thereby enabling the trainee to practice the gait. In this case, the controller 40 regards the torque value of the motor 312b transmitted from the motor driver 313 as the second torque value of the motor 312b under the load condition.

FIG. 7A shows the torque value of the motor 312b over time under the load condition, estimated by the controller 40 according to an embodiment of the disclosure, in which the second torque value of the motor 312b is calculated over time. The motor driver 313 calculates the second torque value of the motor 312b for each gait cycle in real time during the gait training.

While the torque values are divided in units gait cycles and averaged under the no-load condition, the torque values are divided for each gait cycle in real time under the load condition and calculated as the torque value of the motor 312b corresponding to each gait cycle, i.e., the second torque value.

The controller 40 retrieves the first torque value corresponding to the current gait training mode among the reference values stored in the memory and compares the retrieved first torque value with the second torque value to estimate the anteroposterior forces currently exerted on each of the footplates 10 (S23).

The controller 40 controls the first torque value and the second torque value to synchronize with the gait cycle, calculates a first difference value between the first torque value and the second torque value in the synchronized gait cycle, and estimates the anteroposterior forces exerted on the footplates 10 by the foot in that gait cycle based on the first difference value.

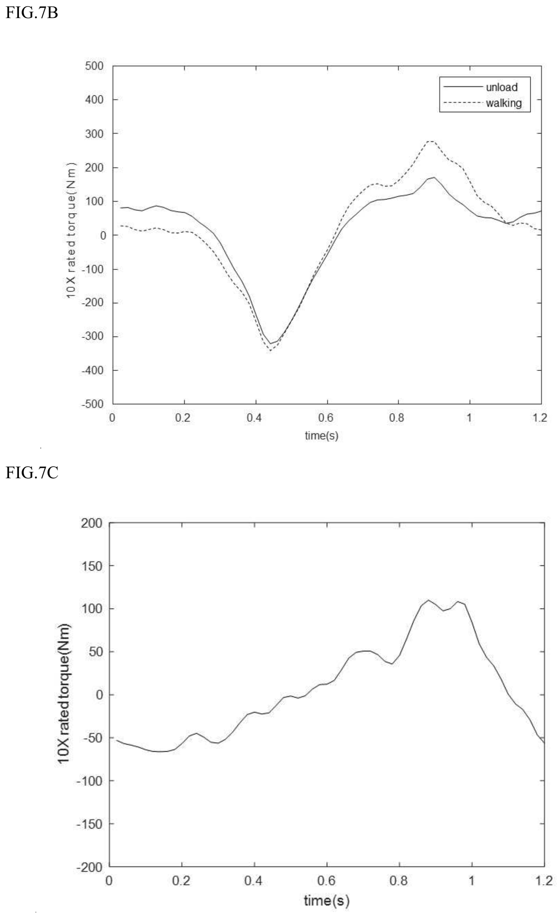

FIG. 7B shows the first torque (solid line) under the no-load condition and the second torque (dotted line) under the load condition, and FIG. 7C shows a difference between the first torque and the second torque.

The controller 40 may continuously monitor the torque value of the motor 312b during the gait training and calculate a difference value in the torque value of the motor 312b between the load condition and the no-load condition in units of gait cycles based on the gait cycle data, thereby calculating the anteroposterior forces exerted on the reference-side footplate 10 and the training-side footplate 10, i.e., the anterior force and the anteroposterior forces.

As described above, when the gait training program includes multiple gait training modes having multiple different gait cycles or gait trajectories, the controller 40 extracts the first torque of the no-load condition corresponding to the gait trajectory and speed according to the currently selected gait training mode from the memory and compares the extracted first torque with the currently estimated second torque value, thereby calculating the anteroposterior forces exerted on the footplate 10.

The controller 40 compares the anteroposterior forces respectively exerted on the reference-side footplate 10 and the training-side footplate 10 (S25), generates training feedback information about the gait training status based on the differences between the compared values, and provides the training feedback information to the trainee (S27).

The reference-side footplate 10 refers to a footplate on which a healthy foot (e.g., non-paretic leg) is placed, and the training-side footplate 10 refers to a footplate on which an unhealthy foot(e.g., a paretic leg) is placed. Through the user interface 70, a user may set the reference-side and training-side footplates in a pair of footplates 10.

The controller 40 compares the absolute values of the anteroposterior forces exerted on the reference-side footplate 10 in the first step with that exerted on the training-side footplate 10 in the second step. When the force exerted on the training-side footplate 10 relative to the force exerted on the reference-side footplate 10 falls within the target range, it is determined that the gait training is being performed normally. On the other hand, when the force exerted on the training-side footplate 10 falls outside the target range, it is determined that the gait training is being performed abnormally.

As described above, the target range of the anteroposterior forces exerted on the training-side footplate 10 may be set by a user through the second UI menu.

Meanwhile, without setting the target section, the controller 40 may determine that the trainee undergoes gait training normally when the force exerted on the reference-side footplate 10 and the force exerted on the training-side footplate 10 are symmetrical.

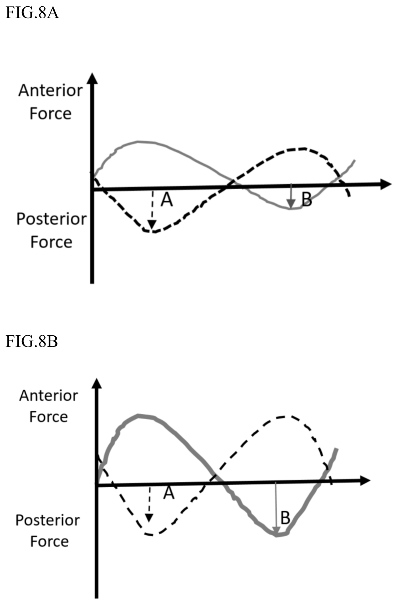

FIGS. 8A and 8B show anteroposterior forces exerted on the left and right footplates 10 in a gait training robot according to an embodiment of the disclosure.

Referring to FIGS. 8A and 8B, the solid lines represent the anteroposterior forces of the training-side footplate 10, and the dotted lines represent the anteroposterior forces of the reference-side footplate 10. Because both feet alternate in stepping during the gait training, the directions of the anteroposterior forces appear in a crossed pattern.

In the following, it will be assumed that the target range of the forces exerted on the training-side footplate 10 is set to 90~100%.

In FIG. 8A, the absolute value B of the force exerted on the training-side footplate 10 in the current step relative to the absolute value A of the anteroposterior forces exerted on the reference-side footplate 10 in the previous step does not reach 90% of the target range, and thus the controller 40 determines that the gait training is not in a normal state.

Similarly, in FIG. 8B, the absolute value B of the force exerted on the training-side footplate 10 is nearly identical to the absolute value A of the anteroposterior forces exerted on the reference-side footplate 10 in the previous step and falls within the target range of 90% to 100%, and thus the controller 40 determines that the gait training is in the normal state.

The controller 40 determines that the training is performed normally when the anteroposterior forces exerted on the training-side footplate 10 relative to the anteroposterior forces exerted on the reference-side footplate 10 fall within a preset target range. Without setting the target range, the controller 40 may determine that the gait training is performed normally when the anteroposterior forces exerted on the training-side footplate 10 fall within a range of 90 to 100% compared to those exerted on the reference-side footplate 10.

The controller 40 may generate and provide training feedback information regarding the trainee's gait training status based on the anteroposterior forces exerted on the reference-side footplate 10 and the training-side footplate 10. For example, the controller may provide at least one of: visual feedback via a display 50, audio feedback via a speaker, or haptic feedback via a vibration sensor. Thus, a trainee or a trainer can determine whether the gait training in each step falls within the set target range and is being performed normally. When the training does not meet the target range, the trainee may be guided to apply more force to his/her training-side foot to undergo the training correctly. Furthermore, when the timing of applying the force to the training-side footplate 10 is incorrect, the controller 40 generates and outputs a timing audio signal, thereby allowing a user to correctly guide the timing of applying the force to the training-side foot.

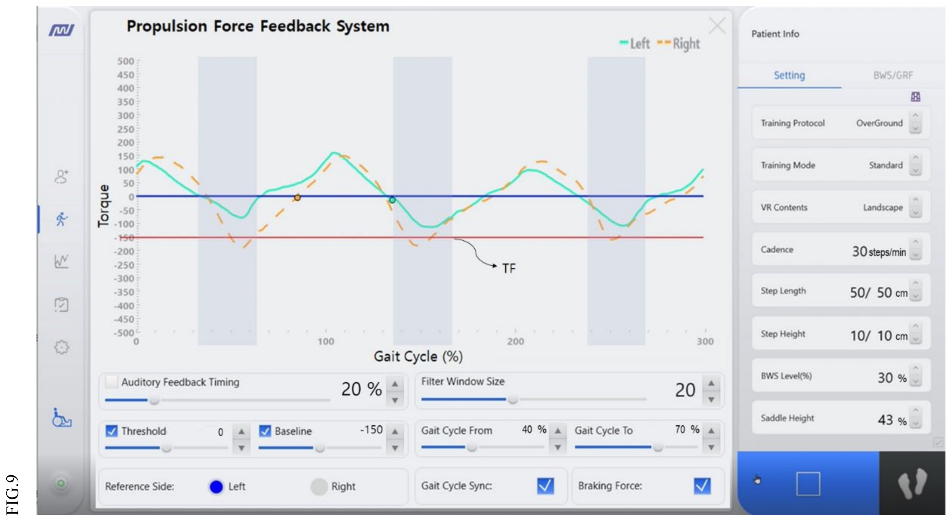

FIG. 9 shows an example of a feedback screen displaying a gait training status of a gait training robot according to an embodiment of the disclosure. Referring to FIG. 9, the anteroposterior forces exerted on the reference-side and training-side are displayed in synchronization with the gait cycles, thereby enabling a more intuitive understanding of the differences between the reference-side and training-side anteroposterior forces. Furthermore, the anteroposterior forces may be numerically represented to provide more accurate data. In FIG. 9, the vertical axis of the graph represents torque, and the horizontal axis represents the gait cycle. The graph illustrates a target value (TF) of the anteroposterior force (e.g., -150) that has been set. The lower left portion of the feedback screen includes several UI elements for setting the reference leg(foot), the target value of the anteroposterior force, and/or the timing of the audio feedback, while the right side includes UI elements for configuring gait training settings.

In the case where the trainee is a hemiplegic patient, the reference-side and training-side anteroposterior forces are compared during gait training, and visual and/or auditory feedback is provided to generate greater anteroposterior forces on the training side, thereby improving the efficiency and effectiveness of training.

Furthermore, when the difference between the reference-side and the training-side anteroposterior forces continues to fall outside the target range, or when the anteroposterior forces exerted on the training-side footplate fails to satisfy a set target value, the controller may change the current gait training mode into another gait training mode having a different training level or correct at least one of the gait trajectory and speed for at least one of the reference-side footplate and the training-side footplate. In this way, the controller may enter an appropriate gait training mode according to the trainee's training status, thereby enabling a trainee to undergo more appropriate gait training.

Meanwhile, in another embodiment of the present invention, virtual-reality-based visual, auditory, and/or haptic feedback may be provided. For example, by using a software program that provides a virtual reality (VR) environment and devices such as a head-mounted display (HMD) or a display screen, visual feedback may be represented during gait training by changing VR-based imagery or the speed of the background according to the anteroposterior forces of the reference-side and training-side footplates, thereby facilitating the trainee's understanding of the training situation. For example, the virtual-reality-based feedback information may include feedback information corresponding to a plurality of preset training states, and during gait training the system may generate and provide VR-based feedback information corresponding to one of the plurality of training states according to the difference between the anteroposterior forces of the reference-side and training-side. For example, the invention may classify training status into multiple levels based on the difference between the anteroposterior forces generated by the reference-side and training-side legs, or based on the degree to which the anteroposterior force generated by the training-side leg satisfies a target range or target value. For example, the controller 40 may compare the anteroposterior force of the training-side leg with that of the reference-side leg or with a target value and classify the training state into seven levels - very deficient, deficient, slightly deficient, normal, slightly excessive, excessive, and very excessive - (alternatively, five-level or three-level schemes such as deficient/normal/excessive may be employed). The controller 40 may then generate and provide VR feedback information corresponding to the determined training state level.

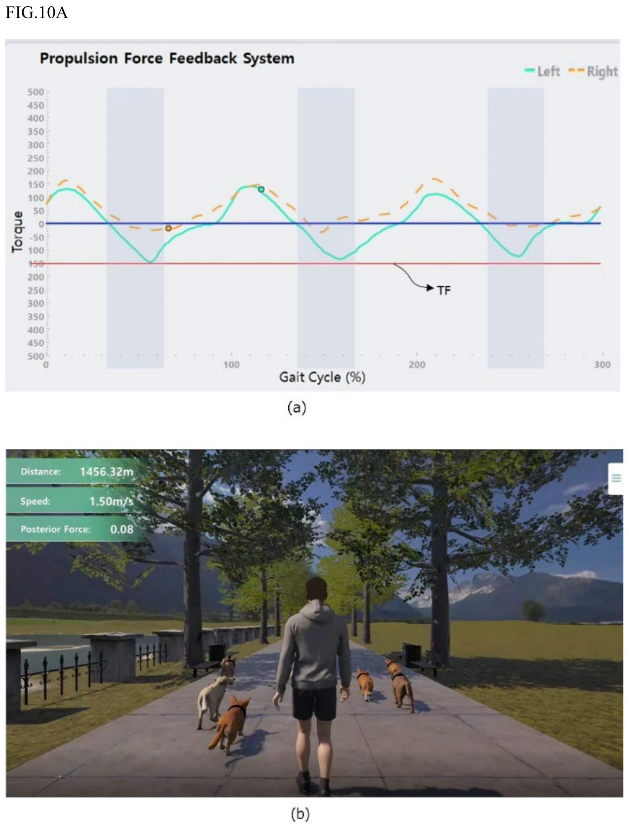

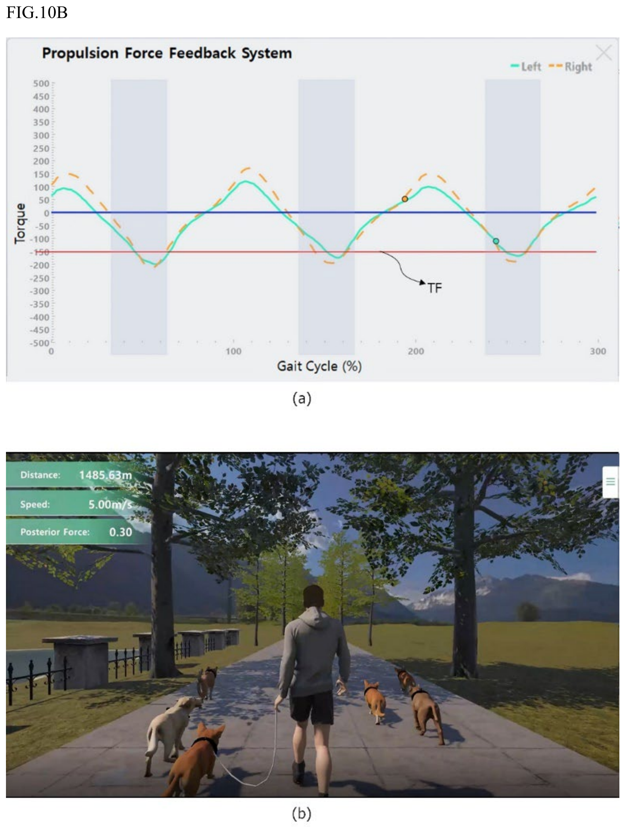

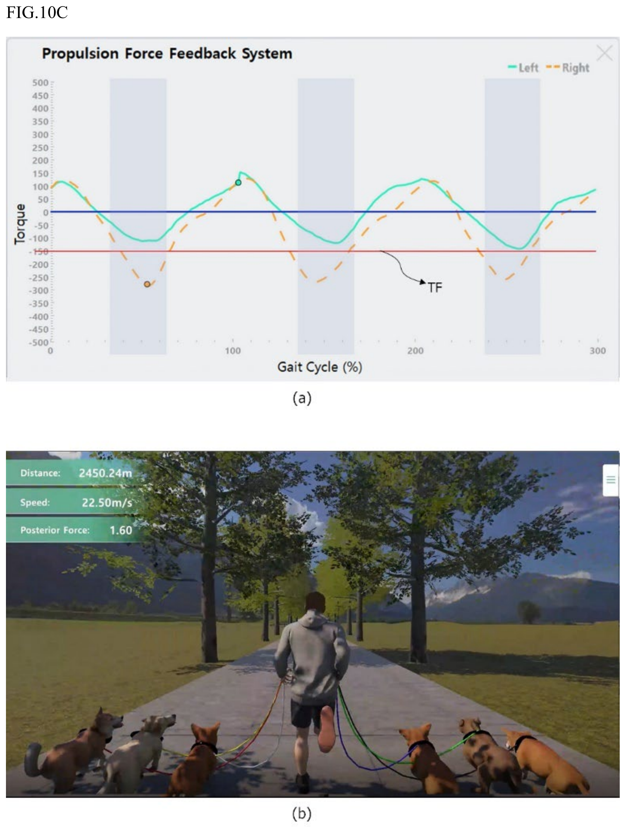

FIGS. 10A, 10B and 10C show three examples of a feedback screen displaying a gait training or propulsion force feedback status of a gait training robot according to a visual reality-based embodiment of the disclosure. In FIGS. 10A, 10B, and 10C, each (a) shows only the graph portion of the feedback screen of FIG. 9, like FIG. 9, shows the torque value on the vertical axis and shows the gait cycle on the horizontal axis. Each feedback screen in (a) shows the torque value wave of the training-side leg (e.g., the paretic leg side) and the torque value wave of the reference-side leg (e.g., non-paretic side leg) over the duration of the gait cycle. The feedback screen also indicates the value of the set target force (TF), allowing the user to check whether the target force is being achieved. For convenience of explanation, only the graphs are illustrated in FIGS. 10A to 10C (a); however, the feedback screen may have the same configuration as that shown in FIG. 9.

In FIGS. 10A, 10B, and 10C, each (b) represents virtual reality-based feedback information corresponding to the respective illustration (a). for example, each feedback screen shows an avatar in shorts and a hoodie walking with multiple dogs on a walking path. In the upper left-hand corner of the right side of each feedback screen, three values are displayed: distance, speed and posterior force. "Distance" indicates the number of meters walked by the trainee; "Speed" indicates the present speed in meters per second of the trainee; and "Posterior Force" indicates the posterior forces exerted by the trainee. For convenience of explanation, the feedback screen is illustrated separately as (a) and (b); however, it should be understood that (a) and (b) may be arranged together in a single screen, either side by side or vertically.

In FIG. 10A, the feedback screen (a) reflects that the trainee is not achieving the target force (-150), which is similar to the posterior force of the reference-side footplate. The controller 40 generates and displays, on the virtual-reality-based feedback screen (b), a scene in which an avatar is not holding a leash and the dogs are walking significantly ahead of the avatar. Such a feedback screen reflects propulsive asymmetry or an abnormal gait that encourages the trainee to generate more force with the paretic leg. In summary, when the posterior force of the training-side leg falls short of the target force (TF), the lack of propulsive force can be represented in the virtual reality environment by depicting the dogs running ahead of the person without leashes.

In FIG. 10B, the feedback screen (a) reflects that the trainee is achieving the target force (-150). Accordingly, the virtual-reality-based feedback screen (b) shows the avatar holding one leash to a dog beside the avatar while two other dogs are walking ahead of the avatar. The virtual-reality-based feedback screen reflects propulsive symmetry or a normal gait that confirms the trainee is generating sufficient force with the paretic leg.

**In** FIG. 10C, the feedback screen (a) reflects that the trainee is achieving the target force (-150) by greater than two times the target force. The torque value wave for the paretic leg dips below -300, which is two times the target force of -150. Accordingly, the virtual reality side of the feedback screen (b) shows the avatar jogging while holding multiple leashes to dogs beside the avatar without any dogs walking ahead of the avatar. The feedback screen reflects propulsive symmetry or an excellent gait that confirms the trainee is strongly exceeding the target force for the paretic leg. In summary, when the posterior force of the training-side leg greatly exceeds the target force (e.g., by approximately twice), multiple leashes are displayed, and the virtual reality scene depicts the person holding all the leashes and running ahead of the dogs, representing the generation of a very strong propulsive force.

The feedback screen, which can be described as a virtual reality-based propulsive force feedback interface, provides the trainee with a more intuitive understanding of the trainee's gait status by providing visualization of the posterior forces in real time within a virtual environment.

In the foregoing embodiment, the torque value (first torque value or second torque value) is calculated from the current value of the motor 312b to estimate the anteroposterior forces exerted on the footplate 10. However, it should be noted that the anteroposterior forces may be estimated using the current value or torque value obtained by various methods described above.

In the foregoing embodiment, the anteroposterior forces are estimated based on the difference in the torque of the motor 312b between the no-load condition and the load condition. However, it should be noted that the anteroposterior forces may be estimated without a new measurement by storing the pattern of the torque of the motor 312b under the no-load condition.

In the foregoing embodiment, the torque from the motor driver 313 is used to estimate the anteroposterior forces. However, it should be noted that the controller of the robot employs the feedback controller as a proportional-integral-derivative controller, and thus the anteroposterior forces are estimated using the current command value and the torque command value based on a position error caused by the anteroposterior forces exerted by a trainee. Alternatively, by comparing the differences between the current command or force command generated from the controller under the load condition and the current command or torque command calculated by a robot model known in advance, it may be estimated that the differences are caused by the anteroposterior forces generated by a trainee.

In the foregoing embodiment, the controller 40 receives the state value or command value of the motor 312b from the motor driver 313. However, the controller 40 may receive the state value of the motor 312b from the current sensor and the encoder and calculate the torque value or control command value of the motor 312b based on the received state value. The state estimation or control of the motor 312b may be performed by the motor driver 313 and/or the controller 40.

As described above, the gait rehabilitation robot according to the disclosure and the control method thereof can estimate the anteroposterior forces exerted on the footplate based on the difference in the torque value of the motor driver between the no-load condition and the load condition, thereby determining the gait status. A training method of making the training-side anteroposterior forces correspond to the reference-side anteroposterior forces may provide propulsion enhancement training.

The disclosure may be implemented by various computer-readable recording media, such as a magnetic storage medium, an optical readout medium, and a digital storage medium, in which a computer program for performing the method according to the disclosure is stored, when executed on a computer. Further, the subject matters expressed as steps in the appended claims are not limited to that order of steps.

Although a few embodiments of the disclosure have been described so far, a person having ordinary knowledge in the art can understand that those embodiments are partially modified or replaced without departing from the technical spirit of the disclosure. Therefore, the scope of the disclosure should be regarded as affecting the subject matters described in the appended claims and its equivalents.

## Claims

1. A gait rehabilitation robot comprising:
a pair of footplates configured to place left and right feet of a trainee thereon for gait training;
a pair of footplate supports, each connected to a corresponding footplate;
a pair of gait actuators configured to actuate respective footplates and respective footplate supports, each gait actuator comprising a motor for moving the corresponding footplate in anterior and posterior directions, and a motor driver for controlling the motor;
a controller configured to control the pair of gait actuators to move and rotate the respective footplates based on a preset gait trajectory and speed; and
a memory configured to store reference values for at least one of state and control of each of the motors according to the preset gait trajectory and speed,
wherein the controller receives at least one of a state value and a control command value relating to the corresponding motor from each gait actuator under a load condition, and calculates anteroposterior forces exerted on each of the footplates by the corresponding foot during the gait training based, for each gait actuator, on a difference between at least one of the received state value and control command value and the corresponding reference value stored in the memory for a present gait trajectory and speed.

2. The gait rehabilitation robot of claim 1, wherein
the reference values comprise at least one of a current value, a torque value, a position value, a speed value, a current command value, a force command value, a position command value, and a speed command value of the motor under a no-load condition,
the state value comprises at least one of a current value, a torque value, a position value, and a speed value of the motor, and
the command value comprises at least one of a current command value, a position command value, a speed command value, and a torque command value.

3. The gait rehabilitation robot of claim 1, wherein
each gait actuator further comprises a current sensor configured to sense a current of the motor,
the reference values of the motor comprise a first current value of the motor measured by the current sensor under the no-load condition or a first torque value calculated based on the first current value,
the state value comprises a second current value of the motor measured by the current sensor under the load condition or a second torque value calculated based on the second current value, and
the controller calculates the anteroposterior forces exerted on each of the footplates by the corresponding foot, based on a difference between the second current value or second torque value of the corresponding motor under the load condition and the first current value or first torque value of the corresponding motor under the no-load condition.

4. The gait rehabilitation robot of claim 1, wherein
the reference values of the motor comprise a first current command value or first torque command value applied by the motor driver under the no-load condition,
the command value comprises a second current command value or second torque command value applied by the motor driver under the load condition, and
the controller calculates the anteroposterior forces exerted on each of the footplates by the corresponding foot, based on a difference between the second current command value or second torque command value of the corresponding motor under the load condition and the first current command value or first torque command value of the corresponding motor under the no-load condition.

5. The gait rehabilitation robot of claim 1, wherein
each gait actuator further comprises an encoder connected to the motor and configured to detect a position and speed of the motor,
the reference values of the motor comprise a first position value or first speed value of the motor under the no-load condition,
the state value comprises a second position value or second speed value of the motor, calculated by the encoder under the load condition, and
the controller calculates the anteroposterior forces exerted on the footplate by the feet, based on a difference between the first position value or first speed value and the first position value or second position value.

6. The gait rehabilitation robot of claim 1, wherein
the controller generates and provides training feedback information related to a gait training status of the trainee based on comparison between the anteroposterior forces of a reference-side footplate in a first step and a training-side footplate in a second step, and
the training feedback information comprises at least one of visual feedback information, auditory feedback information, and tactile feedback information.

7. The gait rehabilitation robot of claim 1, wherein the reference values comprises state values or control values of the motor acquired according to a plurality of different gait trajectories and a plurality of different speeds by controlling each gait actuator to operate along the plurality of different gait trajectories and at the plurality of different speeds under the no-load condition, or comprises a control value of state value of the motor determined by a robot model.

8. The gait rehabilitation robot of claim 1, wherein the controller synchronizes at least one of the state value and command value of each motor with the reference values according to gait cycles, and calculates differences between the at least one of the synchronized state value and command value and the reference values throughout the gait cycle.

9. The gait rehabilitation robot of claim 1, further comprising a user interface (UI) generator configured to generate a first UI menu for setting a target range of anteroposterior forces exerted on a training-side footplate by the trainee compared to the anteroposterior forces exerted on a reference-side footplate,
wherein the controller compares absolute values of anteroposterior forces exerted on the reference-side footplate in a first step with absolute values of anteroposterior forces exerted on the training-side footplate in a second step, and determines that the gait training of the trainee is in a normal state upon the anteroposterior forces exerted on the training-side footplate falling within the target range, but determines that the gait training is in an abnormal state upon the anteroposterior forces exerted on the training-side footplate falling outside the target range.

10. The gait rehabilitation robot of claim 1, further comprising:
a user interface (UI) generator configured to generate a first UI menu for setting a target range of anteroposterior forces exerted on a training-side footplate by the trainee compared to the anteroposterior forces exerted on a reference-side footplate; and
a display,
wherein the controller controls the UI generator to generate a second UI menu that represents the magnitudes of anteroposterior forces exerted on the reference-side footplate and the training-side footplate with respect to a target range and to display the second UI menu on the display.

11. The gait rehabilitation robot of claim 1, further comprising:
a user interface (UI) generator configured to generate a first UI menu for setting a target value of anteroposterior forces exerted on the training-side footplate by the trainee compared to the anteroposterior forces exerted on the reference-side footplate;
wherein the training feedback information includes virtual reality-based feedback information corresponding to each
wherein the controller is further configured to calculate a difference between the anteroposterior forces exerted on the training-side footplate and the target value, and to generate and provide virtual reality-based feedback information corresponding to one of the plurality of the training states based on the calculated difference.

12. The gait rehabilitation robot of claim 10, further comprising a loudspeaker,
wherein the controller generates an audio feedback signal that indicates timing of the anteroposterior forces to be exerted on the training-side footplate and controls the loudspeaker to output the audio feedback signal, upon the anteroposterior forces exerted on the training-side footplate falling outside the target range or upon timing of the anteroposterior forces exerted on the training-side footplate being incorrect.

13. The gait rehabilitation robot of claim 1, further comprising:
a user interface (UI) generator configured to generate a UI menu for setting a target value of anteroposterior forces exerted on the training-side footplate by the trainee;
wherein the controller corrects at least one of a gait trajectory and speed for at least one of the reference-side footplate and the training-side footplate based on a difference in the anteroposterior forces between the reference-side footplate and the training-side footplate or a difference between the anteroposterior forces of the training-side footplate and the target value.

14. A gait rehabilitation robot comprising:
a pair of footplates configured to place left and right feet of a trainee thereon for gait training;
a pair of footplate supports, each connected to a corresponding footplate;
a pair of gait actuators configured to actuate respective footplates and respective footplate supports, each gait actuator comprising a motor for moving the corresponding footplate in anterior and posterior directions, an encoder connected to the motor and detecting position and speed of the motor, and a motor driver for controlling the motor;
a controller configured to control the pair of gait actuators to move and rotate the respective footplates based on a preset gait trajectory and speed; and
a memory configured to store reference values for position or speed of the motor according to the preset gait trajectory and speed,
wherein the motor driver calculates a feedback command value based on an error by comparing a position value or speed value of a corresponding motor calculated by the encoder under the load condition with the reference values, and applies the feedback control value to the corresponding motor, and
the controller calculates anteroposterior forces exerted on each of the footplates by the corresponding foot during the gait training based on the feedback command value.

15. A training method of a gait rehabilitation robot comprising a pair of footplates configured to place left and right feet of a trainee thereon for gait training, and a pair of motors for moving each of the pair of footplates in anterior and posterior directions, the training method comprising:
storing, for each motor, reference values for at least one of a control or a state of the motor based on a preset gait trajectory and speed;
acquiring at least one of a state value and control command value of each motor under a load condition; and
calculating anteroposterior forces exerted on each footplate by the corresponding foot during the gait training, based, for each motor, on a difference between at least one of the state value and command value and the corresponding reference values corresponding to a present gait trajectory and speed.
